(19) Europäisches Patentamt
European Patent Office
Office européen des brevets

(11) Veröffentlichungsnummer: **0 245 315 B1**

(12) **EUROPÄISCHE PATENTSCHRIFT**

(45) Veröffentlichungstag der Patentschrift: **11.04.90**

(51) Int. Cl.$^5$: **C 07 C 45/66**, C 07 C 45/67, C 07 C 49/707, C 07 C 49/753

(21) Anmeldenummer: **86906259.6**

(22) Anmeldetag: **13.11.86**

(86) Internationale Anmeldenummer: **PCT/CH86/00157**

(87) Internationale Veröffentlichungsnummer: **WO 87/03287 04.06.87 Gazette 87/12**

# (54) VERFAHREN ZUR HERSTELLUNG VON -g(a)-HYDROXYCARBONYLVERBINDUNGEN.

(30) Priorität: **20.11.85 CH 4942/85**
**23.09.86 CH 3805/86**

(43) Veröffentlichungstag der Anmeldung: **19.11.87 Patentblatt 87/47**

(45) Bekanntmachung des Hinweises auf die Patenterteilung: **11.04.90 Patentblatt 90/15**

(84) Benannte Vertragsstaaten: **AT BE CH DE FR GB IT LI NL**

(56) Entgegenhaltungen:
**EP-A-0 080 600**
**CH-A- 576 411**
**DE-A-2 360 679**
**FR-A-2 347 329**
**GB-A-2 041 935**
**US-A-2 387 587**
**US-A-3 180 893**
**US-A-3 405 163**

**Chemical Abstracts, Org. Chem. Sect., Band 92, Nr. 9, März 1980 (Columbus, Ohio, USA), Seite 629, Zusammenfassung Nr. 75957a; & JP-A-79103842**

(73) Patentinhaber: **L. GIVAUDAN & CIE Société Anonyme**
**CH-1214 Vernier-Genève (CH)**

(72) Erfinder: **Wild, Hans-Jakob, Dr.**
**Sonnenbergstrasse 13a**
**CH-8633 Wolfhausen (CH)**

(74) Vertreter: **Urech, Peter, Dr. et al**
**Grenzacherstrasse 124 Postfach 3255**
**CH-4002 Basel (CH)**

**EP 0 245 315 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein neues Verfahren zur Herstellung von α-Hydroxycarbonylverbindungen, insbesondere solchen der Formel

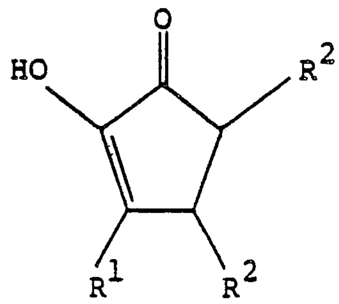

I

worin $R^1$ $C_{1-5}$-Alkyl, insbesondere Methyl, Aethyl, Propyl oder Isopropyl bedeutet und die Radikale $R^2$ unabhängig voneinander Wasserstoff oder $C_{1-5}$-Alkyl, insbesondere Wasserstoff oder Methyl, Aethyl, Propyl oder Isopropyl darstellen.

Das erfindungsgemässe Verfahren ist dadurch gekennzeichnet, dass man eine Verbindung der allgemeinen Formel

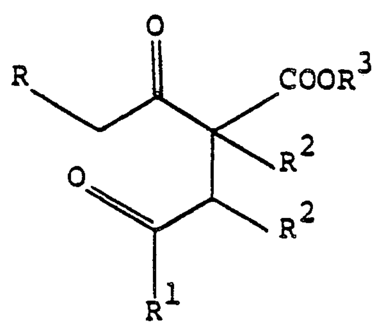

II

worin R für $C_{1-4}$-Alkoxy, Chlor, Brom oder $C_{1-4}$-Alkanoyloxy, z.B. Acetoxy, Propionyloxy, Butyryloxy steht, $R^1$ und $R^2$ obige Bedeutung besitzen und $R^3$ $C_{1-4}$-Alkyl darstellt,
hydrolysiert und einer Aldolkondensation unterwirft.

Bei dieser alkalischen Hydrolyse werden die in II vorhandenen Esterbindungen (R in II = $C_{1-4}$-Alkanoyloxy und die Gruppe —$COOR^3$) gespalten. Die Aldolkondensation liefert die Verbindung I.

Im Falle von R = Chlor oder Brom, wird zunächst nur die Gruppe —$COOR^3$ hydrolytisch gespalten und nach erfolgter Aldolkondensation muss demzufolge auch noch der Halogenrest R alkalisch hydrolysiert werden.

Da die Aldolkondensation vorzugsweise alkalisch durchgeführt wird, muss allerdings im Falle von R = $C_{1-4}$-Alkoxy die auf diese Weise zunächst gebildete Verbindung

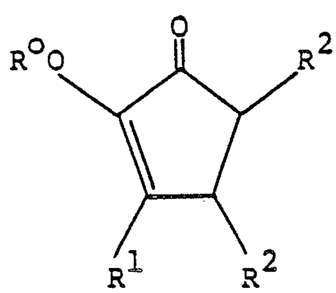

I'

worin $R^0$ für $C_{1-4}$-Alkyl steht und $R^1$ und $R^2$ obige Bedeutung besitzen,
hierauf noch einer — sauren — Aetherspaltung unterworfen werden.

Im Falle von $R^1 \neq R^2 \neq H$ ist Tautomerie möglich. Die Formel I soll demzufolge beide tautomeren Formen und deren Gemische umfassen.

Die zweckmässigen Parameter für das erfindungsgemässe Verfahren, d.h. die erfindungsgemässe

Ueberführung von II in I sind im untenstehenden Reaktionsschema und der dazugehörenden Tabelle I dargestellt.

Dieses Schema und die Tabelle I enthalten zudem die zweckmässigen Parameter des Verfahrens zur Herstellung der Verbindungen II.

In diesem Schema bedeuten:

$R^0$ = $C_{1-4}$-Alkyl, z.B. wie oben angegeben,

Me = Alkalimetall, z.B. Na, K,

AcO = $C_{1-4}$-Alkanoyloxy, z.B. wie oben angegeben,

X = Cl, Br.

Die neuen Verbindungen II werden erhalten, indem man eine Verbindung der Formel

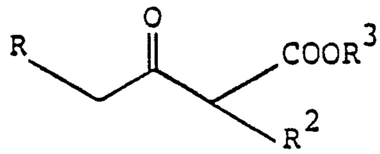

III

worin R, $R^2$ und $R^3$ obige Bedeutung besitzen, alkyliert, zweckmässigerweise mittels Halogencarbonylverbindungen gemäss Reaktionsschema.

In einer besonderen Ausführungsform wird aber im Falle von $R^2$ = $C_{1-5}$-Alkyl ($R^2$ in α-Stellung zur Estergruppe) nicht von einer entsprechenden Verbindung III ausgegangen, sondern es wird die Verbindung der Formel II alkyliert, und zwar vorgängig deren Hydrolyse und der Aldolkondensation, zweckmässigerweise aber, ohne das alkylierte Produkt abzutrennen.

Reaktionsschema

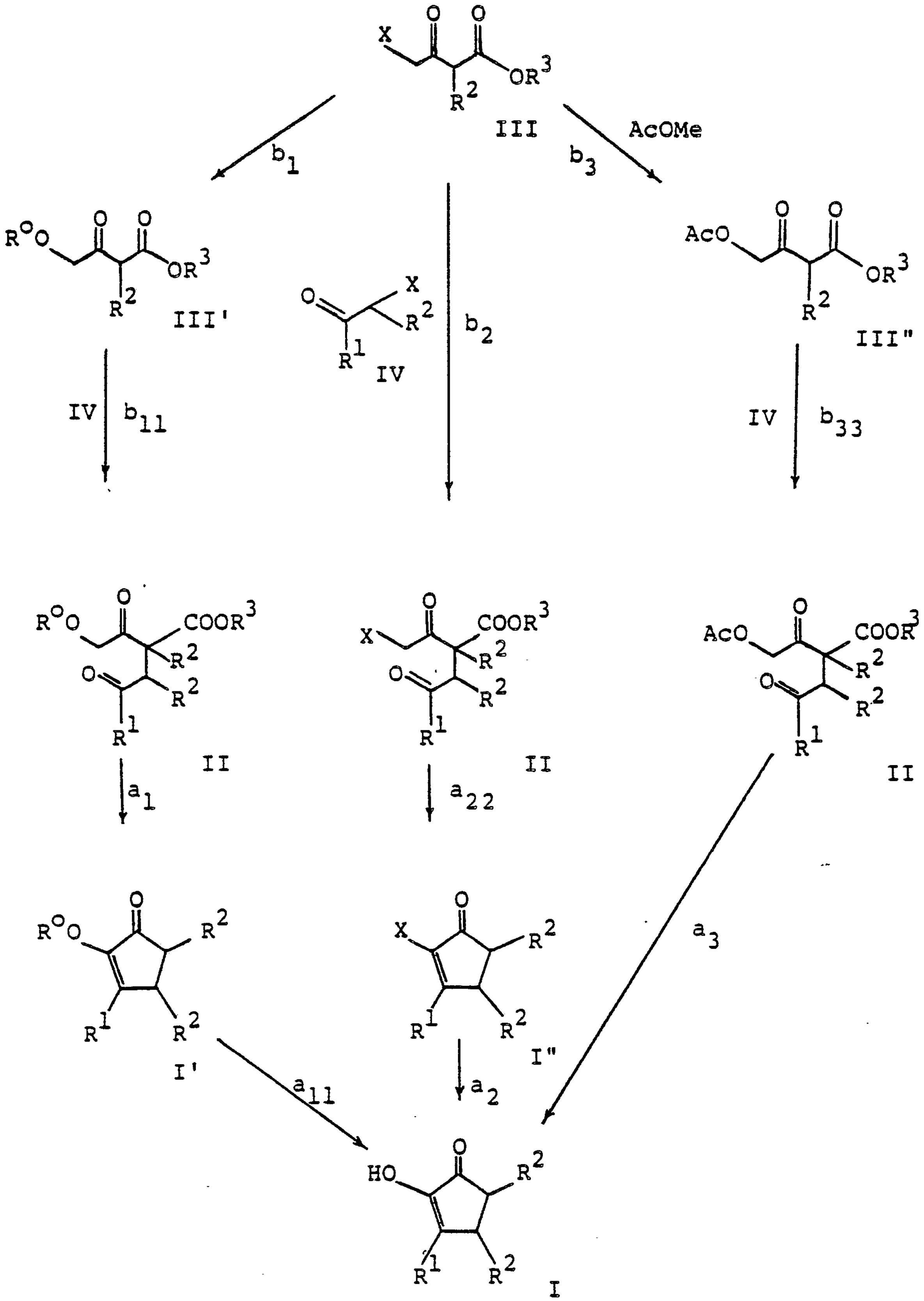


Die Tabelle I informiert über zweckmässige Methoden und Mittel sowie zweckmässige und bevorzugte Reaktionsparameter der einzelnen Verfahrenschritte.

Tabelle I

| Schritt | Reaktionstyp | Mittel | T | Literatur |
|---|---|---|---|---|
| a 1<br>a 3 | Hydrolyse (1) alkalisch (Esterspaltung) zugleich Aldol-kondensation | Metallhydroxyde, Metallcarbonate, Metallbicarbonate in wässriger Lösung | 20-100°C | H.O. House, Modern Synthetic Reactions, 2nd ed. W.A. Benjamin Inc. Menl. Park, California, 1972, 511 seq., 629 seq. |
| a 11 | Enol-Aether-spaltung (1) | starke Säuren z.B. $H_2SO_4$, HCl, $H_3PO_4$<br>wässrige Lösung;<br>HJ, HBr / $CH_3COOH$ | Rückfluss-temperatur | H.O. House, loc. cit. 504 |
| a 2 | Hydrolyse (1) | alkalische Bedingungen: Metallhydroxide, z.B. Alkalimetallhydroxide, Erdalkalimetallhydroxide<br>vorzugsweise in wässriger Lösung, wässrige Ammoniaklösung | 20-100°C | |

Tabelle I

| Schritt | Reaktionstyp | Mittel | T | Literatur |
|---|---|---|---|---|
| a 22 | Hydrolyse (1) alkalisch (Esterspaltung) zugleich Aldolkondensation | schwach basisch: Metallcarbonate, Metallbicarbonate in wässriger Lösung | 20-100°C | H.O. House, loc. cit., 511 seq., 629 seq. |
| b 1 | Substitution (Alkoxylierung) | Alkalialkoholat im Ueberschuss; aprotisches Lösungsmittel, z.B. Acetonitril, Propionitril, etc. | vorzugsweise 50-100°C | EP-PS 76,378 CH-PS 562,191 |
| b 3 | Substitution (Acyloxylierung) | Umsetzung mit Metallalkanoat in polaren aprotischen Lösungsmitteln, z.B. $CH_3CN$, DMF, DMSO, Aceton, Sulfolan, wobei III zweckmässigerweise langsam zum Metallalkanoat zugegeben wird | 50-100°C | Kato et al. J.C.S. Perkin I, (1979), 529 |

(1) nach an sich bekannten Methoden

Tabelle I

| Schritt | Reaktionstyp | Mittel | T | Literatur |
|---|---|---|---|---|
| b 11, b 2, b 33 | Kondensation (Oxalkylierung) | schwach basisches Milieu; insbesondere mittels Alkalicarbonaten oder Alkalibicarbonaten oder mittels Trialkylaminen, z.B. Triäthylamin, vorzugsweise in Anwesenheit von Tetraalkylammoniumsalzen oder Kronenäthern als Katalysatoren<br><br>in polaren Lösungsmitteln, z.B. $CH_3CN$, DMF, DMSO, Aceton, Sulfolan; oder in Wasser; oder auch ohne Lösungsmittel<br><br>III bzw. III' bzw. III": IV = 1:1-3, insb. 1:1-1,5 | 20-100°C | Fedorynski et al., J. Org. Chem. 43 (24), 4682 (1978) |
| | Alkylierung von II (basisch, nicht-wässrig) | Alkylhalogenide/Metallcarbonate, z.B. Alkyliodide/Metallcarbonate, Metallalkoholate in aprotischer Lösungsmitteln, z.B. Acetonitril, DMF, DMSO | 20-80°C | Organikum, organisch-praktisches Grundpraktikum, Autorenkollektiv, VEB, deutscher Verlag der Wissenschaften, Berlin, 1967, Seiten 469ff. |

Die Verbindungen der Formel I sind grösstenteils bekannt Sie stellen Riech- und/oder Geschmackstoffe dar, wobei diesbezüglich die folgenden Verbindungen im Vordergrund des Interesses stehen:

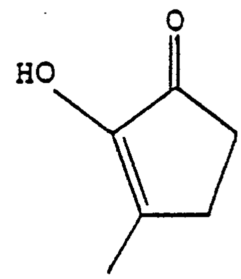
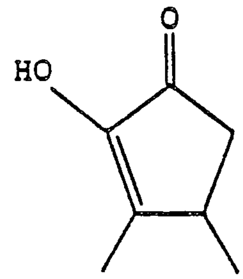
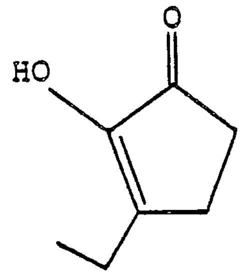

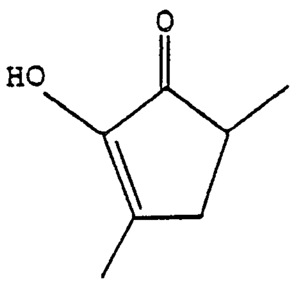
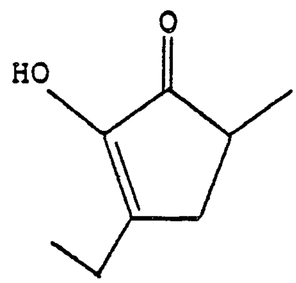
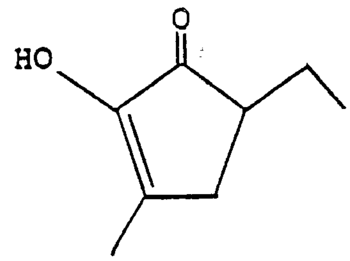

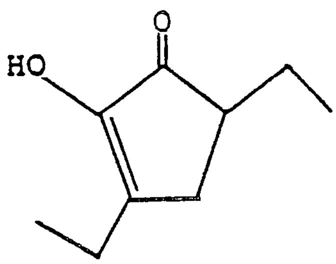
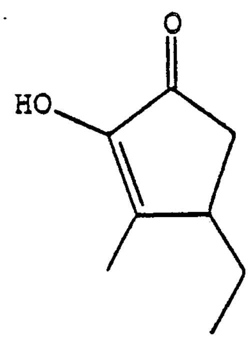
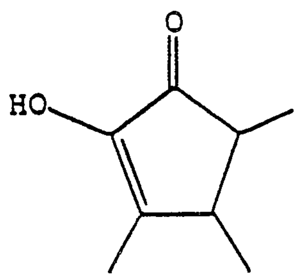
*

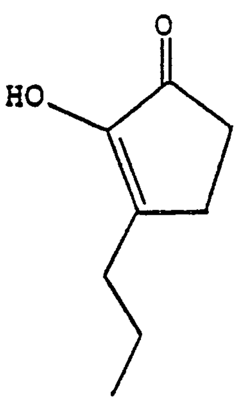
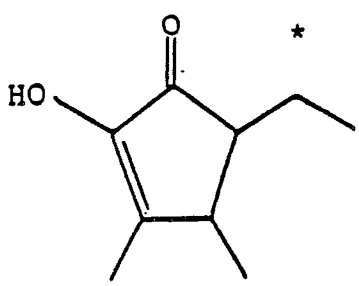
*
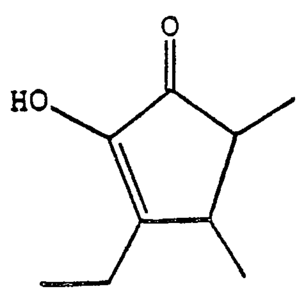
*

Die oben mit Stern bezeichneten Verbindungen sind neu; diese Verbindungen bilden ebenfalls Gegenstand der vorliegenden Erfindung, desgleichen Riech- und/oder Geschmackstoffkompositionen mit

einem Gehalt an den neuen Verbindungen, und schliesslich auch die Verwendung der neuen Verbindungen als Riech- und/oder Geschmackstoffe.

Die organoleptischen Eigenschaften der neuen Verbindungen sind die folgenden:

| | Geruch 1) | Geschmack 2) |
|---|---|---|
| 2-Hydroxy-3,4,5-trimethyl-2-cyclo-penten-1-on | süss, brenzlig kräftig, corylonartig, nach Caramel, Ahorn | süss, brenzlig würzig, corylonartig, nach Caramel, Ahorn Kaffee, Brot, Lakritze |
| 5-Aethyl-2-hydroxy-3,4-dimethyl-2-cyclopenten-1-on + tautomere Form | brenzlig, würzig, kräftig, nach Caramel, Kaffee, Nüssen | sehr ähnlich, wie oben |
| 2-Hydroxy-3,4,4-trimethyl-2-cyclopenten-1-on | zweimal schwächer wie oben | |
| 1) 10% in $C_2H_5OH$ | 2) 3 ppm in $H_2O$ | |

Ein Vergleich des neuen Trimethylderivats mit dem bekanntestem der obigen Aromastoffe, dem 2-Hydroxy-3-methyl-2-cyclopenten-1-on ("Corylone") siehe S. Arctander, Perfume and Flavor Chemicals, Montclair N.J. 1969; 1969 ergab, dass im ersten Fall die Aromenstärke 20 mal höher ist (beide Male in Wasser gemessen, c=1—10 ppm).

Verglichen mit dem bekannten, strukturell verwandten Riechstoff 2-Hydroxy-3,4,4-trimethyl-2-cyclopenten-1-on (Eu-A 80 600) erbringt nur das neue Trimethylderivat in Kompositionen vom Typ Fougère, Rosen, Tabak und Leder überraschende Effekte.

FR—A 2'347 329 beschreibt ein Verfahren zur Herstellung von Verbindungen I mit $R^2$ = H durch Cyclisation von Verbindungen der Formel (2)

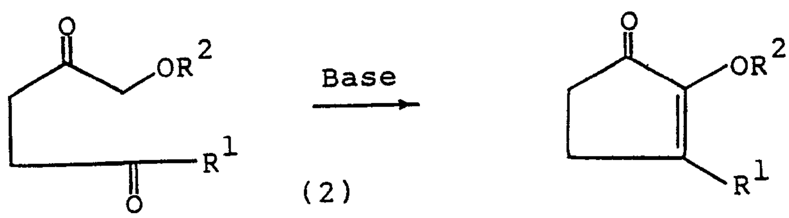


(2) (1)

Die Ausgangsmaterialien selbst sind aus cyclischen Verbindungen zugänglich.

Schliesslich beschreibt die DT—OS 2 360 679 ein Verfahren zur Herstellung von β-Oxo(keto)estern ohne γ-Alkoxy, γ-Chlor, γ-Brom oder γ-Alkanoyloxysubstituenten ausgehend aus α-Halogenketonen und β-Oxyestern.

Solche Verbindungen finden dort Verwendung zur Herstellung von Agrochemikalien.

Die Herstellung von 2-Hydroxy-2-cyclopentenonen ist aus GB—A—2 041 935 bekanntgeworden:

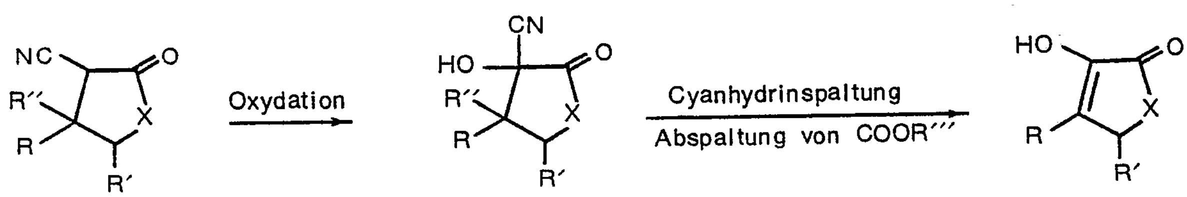


III II I

R = $CH_3$, $C_2H_5$,
R' = H, $CH_3$, $C_2H_5$
X = O, >$CH_2$, >CH=$CH_3$, >$CHC_2H_5$,
R'' = H oder, falls X ≠ O auch COOR''',
R''' = H, $C_{1-6}$ Alkyl.

Die Herstellung von 2-Hydroxy-3-methyl-2-cyclopentenon ist ferner beschrieben in CH—A 576 411. Das

Verfahren ist dadurch gekennzeichnet, dass man Acetonylaceton zu einer siedenden wässrigen Alkalihydroxidlösung gibt, das gebildete 3-Methyl-2-cyclopentenon mit Wasser azeotrop abdestilliert und durch Epoxydation und Spaltung des erhaltenen Epoxids mittels wässrigem Aceton in Gegenwart einer Mineralsäure in 2-Hydroxy-3-methyl-3-cyclopentenon überführt.

Die erfindungsgemässe als Riech- und/oder Geschmackstoffe verwendeten neuen Verbindungen der Formel I veredeln das Geruchsbild von Riechstoffkompositionen, insbesondere von fougèreartigen, chypreartigen, holzigen, animalischen Kompositionen oder Basen. Die Verbindungen I verbinden sich mit zahlreichen bekannten Riechstoffingredienzien natürlichen oder synthetischen Ursprungs, wobei die Palette der natürlichen Rohstoffe sowohl leicht- als auch Mittel- und schwerflüchtige Komponenten, und diejenige der Synthetika Vertreter aus praktisch allen Stoffklassen umfassen kann, wie dies aus der folgenden Zusammenstellung ersichtlich ist:

*Naturprodukte:* Basilikumöl, Baummoos Absolue, Beifussöl, Bergamotteöl, Cassisknospen Absolue, Castoreum, Cedernholzöl, Ciste Labdanum, Civette, Corianderöl, Eichenmoos, Elemiöl, Fichtennadelöl, Galbanum, Geraniumöl, Gewürznelkenöl, Jasmin Absolue und sein synthetischer Ersatz, Jonquille Absolue, Kamillenöl, Labdanum, Lavendelöl, Mandarinenöl, Mastix Absolue, Mentha citrata-öl, Myrrhenöl, Palmarosaöl, Patchouliöl, Petitgrainöl Paraguay, Sandelholzöl, Thymianöl, Vassouraöl, Moschus Infusion, Styrax, Birkenteer, Vetiveröl, Weihrauch, Ylang-Ylang-Oel, Zitronenöl, Zibetöl, etc.

*Alkohole:* Citronellol, Dimetol® (3,6-Dimethyl-heptan-2-ol), Geraniol, cis-3-Hexenol, Linalool, Nonadyl® (6,8-Dimethyl-nonan-2-ol), Phenyläthylalkohol, Rhodinol, Sandela® (3-Isocamphyl-5-cyclohexanol), Sandalore® (3-Methyl-5-(2′,2′,3′-trimethyl-cyclopent-3′-en-1′-yl)-pentan-2-ol), Terpineol, etc.

*Aldehyde:* α-Amylzimtaldehyd, Cyclamenaldehyd, Decanal, Dodecanal, Heliotropin, α-Hexylzimtaldehyd, Hydroxycitronellal, Lyral, Adoxal® (2,6,10-Trimethyl-undec-9-en-1-al), Undecanal, ω-Undecylenaldehyd, Vanillin, etc.

*Ketone:* Isoraldeine® (Isomethyl-α-jonon), α-Jonon, β-Jonon, 3-Prenylisocaranon. Vertofix® (=acetyliertes Cedernholzöl, p-Methylacetophenon, etc.

*Ester:* Acetessigsäureäthylester, Amylsalicylat, Benzylacetat, Cedrylacetat, Cinnamylformiat, Citronellylacetat, Geranylacetat, cis-3-Hexenylacetate, cis-3-Hexenylbenzoat, Linalylacetat, Linalylanthranilat, Methyldihydrojasmonat, Methambrat® (1-Acetoxy-1-methyl-2-sec.-butylcyclohexan), Myraldylacetat® (4(4-Methyl-3-pentenyl-)-cyclohex-3-en-1-yl-carbinylacetat), Phenoxyäthylisobutyrat, Phenyläthyltiglat, Styrallylacetat, Terpenylacetat, 2,3,6,6-Tetramethylcyclohex-2-en-carbonsäure-äthylester, 3,6,6-Trimethyl-2-äthyl-cyclohex-2-en-carbonsäure-äthylester, Vetivenylacetat, ortho-tert. Butylcyclohexylacetat, etc.

*Verschiedene:* Ambrettemoschus, Cumarin, Epoxycedren, Eugenol, Fixolide® (1,1,2,4,4,7-Hexamethyl-6-acetyl-1,2,3,4-tetrahydronaphthalin), Galaxolid® (1,3,4,6,7,8-Hexahydro-4,6,6,7,7,8-hexamethyl-cyclopenta-γ-2-benzopyran), Heliotropin, Indol, Indolen, Isoeugenol, Isobutylchinolin, Jasmonyl(1,3-Diacetoxy-nonan), Ketonmoschus, Limonen, p-Menthan-8-thio-3-on, Madrox® (1-Methylcyclododecyl-methyläther), Methyleugenol, Musk 174® (12-Oxahexadecanolid), γ-Nonalacton, γ-Undecalacton, etc.

Die Verbindungen der Formel I (bzw. deren Gemische) lassen sich in weiten Grenzen einsetzen, die beispielsweise von 0,1 (Detergentien) —30% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,1 und 25%. Die mit I hergestellten Kompositionen lassen sich für alle Arten von parfümierten Verbrauchgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I (bzw. deren Gemische) können demgemäss bei der Herstellung von Kompositionen und — wie obige Zusammenstellung zeigt — unter Verwendung einer breiten Palette bekannter Riechstoff bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Wiese verwendet werden, wie z.B. aus W. A. Poucher, Perfumes, Cosmetics, Soaps *2,* 7. Auflage, Chapman und Hall, London 1974 hervorgehend.

Die neuen Verbindungen der Formel I sind ebenfalls vorzüglich geeignet zur Verwendung in Aromen verschiedenster Art, insbesondere aber auch zur Aromatisierung von Tabak.

Als Geschmackstoffe können die Verbindungen I beispielsweise zur Erzeugung bzw. Verbesserung, Verstärkung, Steigerung oder Modifizierung von Fruchtaromen, z.B. Heidelbeer- oder Brombeeraromen, von Nuss-, Haselnuss-, Mandel-, Schokolade-, Kaffee- und Milcharomen, etc. verwendet werden. Als Anwendungsgebiet dieser Aromen kommen beispielsweise Nahrungsmittel (Joghurt, Süsswaren etc.), Genussmittel (Tee, Tabak, etc.) und Getränke (Limonade etc.) in Frage.

Die ausgeprägten geschmacklichen Qualitäten der Verbindungen I ermöglichen die Verwendung als Aromastoffe in geringen Konzentrationen. Eine geeignete Dosierung umfasst beispielsweise den Bereich von 0,01 ppm — 100 ppm, vorzugsweise den Bereich von 0,01 ppm — 20 ppm im Fertigprodukt, d.h. dem aromatisierten Nahrungsmittel, Genussmittel oder Getränk.

Bei der Aromatisierung von beisielsweise Tabak kann die Dosierung jedoch auch höher liegen und eien grösseren Bereich umfasen, beispielsweise den Bereich von 1 bis 1000 ppm, vorzugsweise 30—100 ppm.

Die Verbindungen können auf übliche Weise mit den für Geschmackstoffkompositionen verwendeten

Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1—10, insbesondere 0,5—3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc., Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin.

## Beispiel 1

a) 113,4 g (2,1 Mol) Natriummethylat werden in 150 ml Acetonitril suspendiert. Man lasst hierauf innert 5 Minuten 150,5 g (1 Mol) Methyl-4-chloracetoacetat zulaufen. Die Temperatur steigt an, wird aber durch Kuhlen bei 68—70°C gehalten. Anschliessend wird noch 25 Minuten bei 70°C nachgerüht. Das Reaktionsgemisch wird auf eine Lösung von 350 ml destilliertes Wasser und 9 g Essigsäure gegossen und durch Zugabe von insgesamt 83 ml 32%-iger Salzsäure bei einem pH-Wert von 6—7 gehalten. Im Scheidetrichter wird die organische Schicht abgetrennt und die wässrige Schicht mit 3 mal je 200 ml Acetonitril extrahiert. Die vereinigten organischen Phasen werden über Magnesiumsulfat getrocknet und am Rotationsverdampfer eingeengt. Das Rohprodukt destilliert bei 55—57°C/0,6 mbar. Es resultieren 132,34 g (90,6%) Methyl-4-methoxyacetoacetat.

IR (fl. Film): $3450^m$, $1750^s$, $1700^s$, $1660^m$ $cm^{-1}$

NMR ($CDCl_3$) 60MHz: 3,44 (s/3H), 3,52 (s/2H), 3,76 (s/3H), 4,10 (s/2H) ppm

MS (m/e): 146 ($M^+$), 115, 101, 59, 45 (100%).

b) 165,8 g (1,2 Mol) Kaliumcarbonat und 4,6 g (0,02 Mol) Benzyltriäthylammoniumchlorid werden in 500 ml Acetonitril suspendiert. Unter Rühren gibt man dazu 146,1 g (1 Mol) Methyl-4-methoxyacetoacetat und tropft innert 10 Minuten 138,8 g (1,5 Mol) Chloraceton zu. Bei 20—25°C wird 7 Stunden weitergerührt. Das Reaktionsgemisch wird auf 1 Liter 10%-ige $NaH_2PO_4$-Lösung/Eis gegossen (pH-Wert = 5) und es wird 3 mal mit je 500 ml Aethylacetat extrahiert. Die vereinigten organischen Phasen werden über Magnesiumsulfat getrocknet, am Rotationsverdampfer eingeengt und ergeben so 206,30 g (100%) Methyl-4-methoxy-2-acetonylacetoacetat, Gehalt: 84,3%; Sdp. 104—105°C/0,06 mbar.

IR (fl. Film): $3610^m$, $1745^s$, $1715^m$, $1630^m$ $cm^{-1}$

NMR ($CDCl_3$) 60MHz: 2,20 (s/3H), 3,01—3,21 (m/2H), 3,45 (s/3H), 3,73 (s/3H), 4,00—4,23 (m/1H), 4,28 (s/2H) ppm

MS (m/e): 202 ($M^+$), 157, 125, 97, 55, 45 (100%).

C) 201,3 g (0,997 Mol) rohes Methyl-4-methoxy-2-acetonylacetoacetat werden mit 2,12 Liter (0,5 Mol) 2,5%-iger $Na_2CO_3$-Lösung während 3 Stunden bei Rückflusstemperatur gehalten. Das Reaktionsgemisch wird 3 mal mit je 500 ml $CH_2Cl_2$ extrahiert. Die vereinigten organischen Phasen werden über Magnesiumsulfat getrocknet, am Rotationsverdampfer eingeengt und ergeben so 99,90 g (79,5%) 2-Methoxy-3-methyl-2-cyclopenten-1-on, Gehalt 84,2%; Sdp. 82—84°C/27 mbar.

IR (fl. Film): $1700^s$, $1645^s$ $cm^{-1}$

NMR ($CDCl_3$) 60MHz: 2,00 (s/3H), 2,40 (s/4H), 3,88 (s/3H) ppm

Ms (m/e): 126 ($M^+$, 100%), 111, 97, 83, 55.

d) 94,8 g (0,75 Mol) rohes 2-Methoxy-3-methyl-2-cyclopenten-1-on werden mit 1,896 kg (20 fache Gewichtsmenge) 5 N Salzsäure während 5 Stunden bei Rückflusstemperatur gehalten. Das Reaktionsgemisch wird 3 mal mit je 300 ml $CH_2Cl_2$ extrahiert. Die organischen Phasen werden 2 mal mit je 500 ml 2 N Natronlauge extrahiert. Die vereinigten NaOH-Auszüge werden unter Kühlen mit konzentrierter Salzsäure auf einen pH-Wert von 5 gestellt und dann 3 mal mit je 300 ml $CH_2Cl_2$ extrahiert. Die vereinigten organischen Phasen werden über Magnesiumsulfat getrocknet und am Rotationsverdampfer eingeengt. Nach Umkristallisation aus Aceton/Wasser 1:1 erhält man 57,66 g (68,4%) 2-Hydroxy-3-methyl-2-cyclopenten-1-on; Smp. 100—102°C.

IR ($CHCl_3$): $3500^m$, $3310^w$ (breit), $1710^s$, $1660^s$ $cm^{-1}$

NMR ($CDCl_3$) 60MHz: 2,02 (s/3H), 2,45 (s/4H), 7,00 (s, breit/1H) ppm

MS (m/e): 112 ($M^+$, 100%), 97, 84, 69, 55, 41.

## Beispiel 2

a) 108 g (1,1 Mol) Kaliumacetat und 11,4 g (0,05 Mol) Benzyltriäthylammoniumchlorid werden in 900 ml

Acetonitril aufgeschlämmt, mit 50 ml Essigsäure versetzt und auf Rückflusstemperatur erhitzt. Innert 3½ Stunden tropft man 150,5 g (1 Mol) Methyl 4-chloracetoacetat in 100 ml Acetonitril zu und hält das Gemisch anschliessend 4 Stunden bei Rückflusstemperatur. Das Reaktionsgemisch wird 3 mal mit je 200 ml 5%-iger $NaH_2PO_4$-Lösung gewaschen und die wässrigen Phasen mit 200 ml $CH_2Cl_2$ zurückextrahiert. Die vereinigten organischen Phasen werden über Magnesiumsulfat getrocknet und am Rotationsverdampfer eingeengt. Das Rohprodukt destilliert bei 76—85°C/0,6 mbar und ergibt 106,17 g (61%) Methyl-4-acetoxyacetoacetat.

IR (fl. Film): 3160—3450$^w$ (komplex), 1740$^s$ (breit), 1650$^m$ (s)

NMR ($CDCl_3$) 60MHz: 2,19 (s/3H), 3,53 (s/2H), 3,78 (s/3H), 4,80 (s/2H) ppm

MS (m/e): 174 ($M^+$), 132, 101, 74, 59, 43 (100%).

b) 63,6 g (0,6 Mol) Natriumcarbonat und 2,3 g (0,01 Mol) Benzyltriäthylammoniumchlorid werden in 250 ml Acetonitril ausgeschlämmt. Unter Rühren gibt man 87,0 g (0,5 Mol) Methyl-4-acetoxyacetoacetat zu und tropft innert 5 Minuten 69,4 g (0,75 Mol) Chloraceton zu. Bei 20—25°C wird 46 Stunden weitergerührt. Das Reaktionsgemisch wird auf 1 Liter 10%-ige $NaH_2PO_4$-Lösung/Eis gegossen (pH-Wert = 5) und 3 mal mit je 300 ml Aethylacetat extrahiert. Die vereinigten organischen Phasen werden über Magnesiumsulfat getrocknet, am Rotationsverdampfer völlig eingeengt und ergeben 105,9 g (92%) Methyl-4-acetoxy-2-acetonylacetoacetat, Gehalt 76,9%; Sdp. 119—121°C/0,06 mbar.

IR (fl. Film): 1730$^s$ (breit, komplex) $cm^{-1}$

NMR ($CDCl_3$) 60MHz: 2,18 (s/3H), 2,21 (s/3H), 3,09—3,22 (m/2H), 3,76 (s/3H), 4,11 (t/1H), 4,97 (s/2H) ppm

MS (m/e): 170, 157, 125, 97, 87, 43 (100%).

c) 230 mg (1 mMol) rohes Methyl-4-acetoxy-2-acetonylacetoacetat werden mit 2 ml (0,19 mMol) 1%-iger $Na_2CO_3$-Lösung während 1 Stunde bei Rückflusstemperatur gehalten, wobei der pH-Wert auf 6 sinkt. Man gibt festes $Na_2CO_3$ zu, bis der pH-Wert auf 11—12 bleibt und hält nochmals 1 Stunde bei Rückflusstemperatur. Das Reaktionsgemisch wird auf 10 ml 10%-ige $NaH_2PO_4$-Lösungs/Eis gegossen (pH-Wert = 5) und 3 mal mit je 20 ml $CH_2Cl_2$ extrahiert. Die vereinigten organischen Phasen werden mit Magnesiumsulfat getrocknet, am Rotationsverdampfer eingeengt und ergeben 60,2 mg (53,7%) 2-Hydroxy-3-methyl-2-cyclopenten-1-on, Gehalt: 95,7% Smp. 100—102°C.

IR ($CHCl_3$): 3500$^m$, 3310$^w$ (breit), 1710$^s$, 1660$^s$ $cm^{-1}$

NMR ($CDCl_3$) 60MHz: 2,02 (s/3H), 2,45 (s/4H) ppm.

## Beispiel 3

a) 127,2 g (1,2 Mol) Natriumcarbonat und 4,6 g (0,02 Mol) Benzyltriäthylammoniumchlorid werden in 800 ml Acetonitril suspendiert. Unter Rühren tropft man innert 8 Stunden eine Lösung von 150,5 g (1 Mol) Methyl-4-chloracetoacetat und 138,8 g (1,5 Mol) Chloraceton in 200 ml Acetonitril zu. Bei 20—25°C wird 15 Stunden nachgerührt. Das Reaktionsgemisch wird auf 750 ml 2 N HCl gegossen, (pH-Wert = 1) und 3 mal mit 300 ml Aether extrahiert. Die vereinigten organischen Phasen werden über Magnesiumsulfat getrocknet und am Rotationsverdampfer eingeengt. Der Rückstand wird mit 200 ml Aether versetzt, das ausgefallene 2,5-Bisäthoxycarbonyl-1,4-cyclohexandion abgenutscht und das Filtrat wieder eingeengt. Das Rohprodukt wird mit Kieselgel in Aether/Hexan 1:1 säulenchromatographiert und ergibt 32,28 g (15,6%) Methyl-4-chlor-2-acetonylacetoacetat.

IR (fl. Film): 3630—3400$^w$ (komplex), 1750$^s$, 1715$^s$, 1660$^m$ $cm^{-1}$

NMR ($CDCl_3$) 60MHz: 2,20 (s/3H), 3,10—3,30 (m/2H), 3,78 (s/3H), 4,12—4,33 (m/1H), 4,50 (s/2H), ppm

MS (m/e): 206 ($M^+$), 175, 157, 125, 97, 87, 77, 49, 43 (100%).

b) 6,20 g (30 mMol) Methyl-4-chlor-2-acetonylacetoacetat werden mit 63,6 g (15 mMol) 2,5%-iger $Na_2CO_3$-Lösung während 1 Stunde bei Rückflusstemperatur gehalten. Das Reaktionsgemisch wird auf 30 ml 1 N HCl gegossen und 3 mal mit 50 ml $CH_2Cl_2$ extrahiert. Die vereinigten organischen Phasen werden über Magnesiumsulfat getrocknet und am Rotationsverdampfer eingeengt. Das Rohprodukt wird mit Kieselgel in Aether/Hexan/$CH_2Cl_2$ 1:1:3 säulenchromatographiert und ergibt 0,80 g (20,4%) 2-Chlor-3-methyl-2-cyclopenten-1-on, Gehalt 93,2%; Smp. 37—39°C.

IR ($CHCl_3$): 1715$^s$, 1625$^s$ $cm^{-1}$

NMR ($CDCl_3$) 60MHz: 2,20 (s/3H), 2,60 (m/4H) ppm

MS (m/e): 130 ($M^+$), 115, 102, 95, 67 (100%).

c) 0,72 g (5,5 mMol) 2-Chlor-3-methyl-2-cyclopenten-1-on werden mit 27,5 ml (27,5 mMol) 1 N Natronlauge während 1 Stunde bei Raumtemperatur gerührt. Das Reaktionsgemisch wird mit konzentrierter Salzsäure auf einen pH-Wert von 3 gebracht und 3 mal mit 20 ml $CH_2Cl_2$ extrahiert. Die vereinigten organischen Phasen werden über Magnesiumsulfat getrocknet und am Rotationsverdampfer eingeengt. Nach Umkristallisation aus Aether/Hexan 1:1 erhält man 0,47 g (76,3%) 2-Hydroxy-3-methyl-2-cyclopenten-1-on; Smp. 101—102°C.

IR ($CHCl_3$): 3500 $^m$, 3310$^w$ (breit), 1710$^s$, 1660$^s$ $cm^{-1}$

NMR ($CDCl_3$) 60MHz: 2,02 (s/3H), 2,45 (s/4H), 5,80 (s, breit/1H) ppm

MS (m/e): 112 ($M^+$, 100%), 97, 84, 69, 55, 41.

## Beispiel 4

a) seihe Beispiel 1 a).

b) 16,58 g (120 mMol) Kaliumcarbonat und 0,46 g (2 mMol) Benzyltriäthylammoniumchlorid werden in 50 ml Acetonitril suspendiert. Unter Rühren gibt man 14,60 g (100 mMol) Methyl-4-methoxyacetoacetat zu

und tropft innert 10 Minuten 15,97 g (150 mMol) 3-Chlor-2-butanon zu. Bei 20—25°C wird 65 Stunden nachgerührt. Das Reaktionsgemisch wird auf 70 ml 4 N HCl/Eis gegossen (pH-Wert = 1) und 3 mal mit je 100 ml Aethylacetat extrahiert. Die vereinigten organischen Phasen werden über Magnesiumsulfat getrocknet und am Rotationsverdampfer eingeengt. Das Rohprodukt destilliert bei 95°C/0,05 mbar und ergibt 6,67 g (42%) Methyl-4-methoxy-2-[1-methylacetonyl]-acetoacetat.

IR (fl. Film): 3620—3400$^{w}$ (breit), 1745$^{s}$, 1715$^{s}$, 1635$^{w}$ $cm^{-1}$

NMR ($CDCl_3$) 60MHz: 1,08 + 1,16 (2d/3H), 2,20 + 2,23 (2s/3H), 3,15—3,55 (m/1H), 3,41 + 3,44 (2s/3H), 3,85—4,06 (m/1H), 4,18 + 4,21 (2s/2H) ppm

MS (m/e): 216 ($M^+$), 171, 139, 111, 69, 45 (100%).

c) 2,16 g (10 mMol) Methyl-4-methoxy-2-[1-methylacetonyl]-acetoacetat werden mit 21,2 ml (5 mMol) 2,5%-iger $Na_2CO_3$-Lösung während 2 Stunden bei Rückflusstemperatur gehalten. Das Reaktionsgemisch wird 3 mal mit je 25 ml $CH_2Cl_2$ extrahiert. Die vereinigten organischen Phasen werden über Magnesiumsulfat getrocknet, am Rotationsverdampfer eingeengt und ergeben 1,40 g (100%) 2-Methoxy-3,4-dimethyl-2-cyclopenten-1-on, Gehalt 96%.

IR (fl. Film): 1700$^{s}$, 1640$^{s}$ $cm^{-1}$

NMR ($CDCl_3$) 60MHz: 1,19 (d/3H), 1,96 (s/3H), 1,72—2,83 (m/3H), 4,91 (s/3H) ppm.

d) 0,70 g (5 mMol) rohes 2-Methoxy-3,4-dimethyl-2-cyclopenten-1-on werden mit 7 g (10-fache Gewichtsmenge) 5 N Salzsäure während 3 Stunden bei Rückflusstemperatur gehalten. Das Reaktionsgemisch wird 3 mal mit je 50 ml $CH_2Cl_2$ extrahiert. Die vereinigten organischen Phasen werden über Magnesiumsulfat getrocknet, am Rotationsverdampfer eingeengt und ergeben so 0,45 g (71,4%) 2-Hydroxy-3,4-dimethyl-2-cyclopenten-1-on, Gehalt 90,6%. Die Daten dieses Materials sind:

IR ($CHCl_3$): 3500$^{m}$, 3310$^{w}$ (breit), 1710$^{s}$, 1660$^{s}$ $cm^{-1}$

NMR ($CDCl_3$) 60MHz: 1,19 (d/3H), 1,99 (s/3H), 1,76—2,83 (m/3H), 6,02 (s, breit/1H).

Die Verbindung kann gemäss Beispiel 1.d) gereinigt werden.

Wo nicht anders vermerkt, erfolgten die Gehaltsbestimmungen gaschromatographisch.

Die IR-, NMR- und MS-Daten wurden — wo nicht anders vermerkt — am gereinigten, beispielsweise durch Kristallisation oder Destillation gereinigten Material ermittelt.

Beispiel 5

a) 1,66 g (12 mMol) Kaliumcarbonat und 0,04 g (0,2 mMol) Benzyltriäthylammoniumchlorid werden in 5 ml Acetonitril suspendiert. Unter Rühren gibt man 1,60 g (10 mMol) Methyl-4-methoxy-2-methylacetoacetat zu und tropft innert 15 Minuten 1,39 g (15 mMol) Chloraceton zu. Bei 20—25°C wird 21 Stunden weitergerührt. Das Reaktionsgemisch wird auf 20 ml 2N HCl gegossen (pH-Wert = 1) und es wird 3 ml mit je 20 ml Aethylacetat extrahiert. Die vereinigten organischen Phasen werden über Magnesiumsulfat getrocknet und am Rotationsverdampfer eingeengt. Das Rohprodukt destilliert bei 95°C/0,03 mbar. Es resultieren 0,83 g (38,4%) Methyl-4-methoxy-2-acetonyl-2-methylacetoacetat.

IR (fl. Film): 1745$^{s}$ (Schulter), 1720$^{s}$, 1630$^{w}$ $cm^{-1}$

NMR ($CDCl_3$) 60MHz: 1,45 (s/3H), 2,12 (s/3H), 3,15 (s/2H), 3,40 (s/3H), 3,71 (s/3H, 4,38 (s/2H) ppm

MS (m/e): 216 ($M^+$), 184, 171, 143, 111, 45, 43, (100%).

b) 5,40 g (25 mMol) Methyl-4-methoxy-2-acetonyl-2-methylacetoacetat werden mit 106 ml (12,5 mMol) 2,5%-iger $Na_2CO_3$-Lösung während 2½ Stunden bei Rückflusstemperatur gehalten. Das Reaktionsgemisch wird 3 mal mit je 150 ml $CH_2Cl_2$ extrahiert. Die vereinigten organischen Phasen werden über Magnesiumsulfat getrocknet, am Rotationsverdampfer eingeengt und ergeben so 2,66 g (76%) 2-Methoxy-3,5-dimethyl-2-cyclopenten-1-on, Gehalt: 96,7%.

IR (fl. Film): 1705$^{s}$, 1650$^{s}$ $cm^{-1}$

NMR ($CDCl_3$) 60MHz: 1,18 (d/3H), 1,98 (s/3H), 1,80—3,00 (m/3H), 3,90 (s/3H) ppm.

c) 2,15 g (15,36 mMol) rohes 2-Methoxy-3,5-dimethyl-2-cyclopenten-1-on werden mit 21,50 g (=10 fache Gewichtsmenge) 5N Salzsäure während 2 Stunden bei Rückflusstemperatur gehalten. Das Reaktionsgemisch wird 3 mal mit je 50 ml $CH_2Cl_2$ extrahiert. Die vereinigten organischen Phasen werden über Magnesiumsulfat getrocknet und am Rotationsverdampfer eingeengt. Nach Umkristallisation aus Aether/Hexan 1:1 erhält man 1,05 g (54,2%) 2-Hydroxy-3,5-dimethyl-2-cyclopenten-1-on; Smp. 92—93°C.

IR ($CHCl_3$): 3500$^{m}$, 3310$^{m}$ (breit), 1710$^{s}$, 1660$^{s}$ $cm^{-1}$

NMR ($CDCl_3$) 60MHz: 1,18 (d/3H), 2,00 (s/3H), 1,80—3,00 (m/3H), 6,40 (s/1H) ppm

MS (m/e): 126 ($M^+$, 100%), 111, 98, 83, 79, 69, 55, 43.

Beispiel 6

a) 15,2 g (110 mMol) Kaliumcarbonat und 0,46 g (2 mMol) Benzyltriäthylammoniumchlorid werden in 100 ml Acetonitril suspendiert. Unter Rühren gibt man 20,2 g (100 mMol) Methyl-4-methoxy-2-acetonyl-acetoacetat hinzu und erwärmt auf 60°C. Innert 100 Minuten tropft man 18,7 g (120 mMol) Aethyljodid zu und rührt das Gemisch noch 24 Stunden bei 60°C weiter. Dann giesst man die Suspension auf eine Lösung von 6,9 g (50 mMol) Kaliumcarbonat in 300 ml Wasser und erhitzt 3 Stunden im Rückfluss. Das abgekühlte Reaktionsgemisch wird 3 mal mit 100 ml Aether extrahiert. Die vereinigten organischen Phasen werden über Magnesiumsulfat getrocknet, am Rotationsverdampfer eingeengt und ergeben so 12,92 g (84%) 5-Aethyl-2-methoxy-3-methyl-2-cyclopenten-1-on, Gehalt (GC): 97%. Siedepunkt 95°C/14 mbar.

IR (fl. Film): 1700$^{s}$, 1645$^{s}$ $cm^{-1}$

NMR ($CDCl_3$) 60MHz: 0,95 (t/3H), 2,02 (s/3H), 1,4—2,9 (m/5H), 3,9 (s)3H) ppm.

b) 12,8 g (83 mMol) rohes 5-Aethyl-2-methoxy-3-methyl-2-cyclopenten-1-on werden mit 128 g 5N Salzsäure während 2 Stunden bei Rückflusstemperatur gehalten. Das abgekühlte Reaktionsgemisch wird 3 mal mit 100 ml $CH_2Cl_2$ extrahiert. Die vereinigten Phasen werden über Magnesiumsulfat getrocknet und am Rotationsverdampfer eingeengt. Das Rohprodukt wird bei 47—48°C/0,03 mbar destilliert und liefert 9,09 g (78,2%) eines Gemisches von 5-Aethyl-2-hydroxy-3-methyl-2-cyclopenten-1-on (Isomer A) und 3-Aethyl-2-hydroxy-5-methyl-2-cyclopenten-1-on (Isomer B) im Verhältnis von ca. 7:3, Gehalt (GC): Isomer A 70,2%. Isomer B 28,8%.

IR ($CHCl_3$): $3500^m$, $3300^m$ (breit), $1705^s$, $1655^s$ $cm^{-1}$

NMR ($CDCl_3$) 400MHz: Isomer A: 0,95 (t/3H), 1,43 (m/1H), 1,82 (m/1H), 2,02 (s/3H), 2,12 (m/1H), 2,35 (m/1H), 2,60 (m/1H), 6,5 (s, breit/1H ppm. Isomer B: 1,15 (t/3H), 1,18 (d/2H), 2,03 (m/1H), 2,43 (m/1H), 2,45 (q/2H), 2,71 (m/1H), 6,5 (s, breit/1H) ppm.

MS (m/e): 140 ($M^+$), 125, 112, 107, 97, 94 (100%).

Beispiel 7

a) 15,20 g (110 mMol) Kaliumcarbonat und 0,46 g (2 mMol) Benzyltriäthylammoniumchlorid werden in 100 ml Acetonitril suspendiert. Unter Rühren gibt man 21,60 g (100 mMol) Methyl-4-methoxy-2-[1-methyl-acetonyl]-acetoacetat zu, erwärmt auf 60°C und tropft innert 30 Minuten 28,39 g (200 mMol) Methyljodid zu. Bei 60°C wird 16 Stunden weitergerührt; dann addiert man nochmals 6,91 g (50 mMol) Kaliumcarbonat und 7,10 g (50 mMol) Methyljodid und rührt weitere 21 Stunden bei 60°C. Nun gibt man 6,90 g (50 mMol) Kaliumcarbonat in 300 ml destilliertem Wasser zu. Das Reaktionsgemisch wird 6 Stunden bei Rückflusstemperatur gehalten. Dann wird 3 mal mit je 100 ml Aether extrahiert. Die vereinigten organischen Phasen werden über Magnesiumsulfat getrocknet und am Rotationsverdampfer eingeengt. Das Rohprodukt destilliert bei 70—78°C/12 mm und liefert 6,03 g (39,2%) 2-Methoxy-3,4,5-trimethyl-2-cyclopenten-1-on.

IR (fl. Film): $1700^s$, $1640^s$ $cm^{-1}$

NMR ($CDCl_3$) 60MHz: 1,15 (d/6H), 1,92 (s/3H), 1,60—2,80 (m/2H), 3,88 (s/3H) ppm

MS (m/e): 154 ($M^+$), 139 (100%), 126, 111, 96, 79, 67, 55, 41.

b) 5,39 g (35 mMol) 2-Methoxy-3,4,5-trimethyl-2-cyclopenten-1-on werden mit 53,9 g (10 fache Gewichtsmenge) 5N Salzsäure während 90 Minuten bei Rückflusstemperatur gehalten. Das Reaktionsgemisch wird 3 mal mit je 50 ml Aether extrahiert. Die vereinigten organischen Phasen werden über Magnesiumsulfat getrocknet und am Rotationsverdampfer eingeengt. Das Rohprodukt wird bei 45—48°C/0,03 mbar destilliert und liefert 3,57 g (72,9%) 2-Hydroxy-3,4,5-trimethyl-2-cyclopenten-1-on.

IR ($CHCl_3$): $3500^m$, $3310^w$ (breit), $1710^s$, $1660^s$ $cm^{-1}$

NMR ($CDCl_3$) 60MHz: 1,15 (d/6H), 1,96 (s/3H), 1,60—2,80 (m/2H), 7,10 (s, breit/1H) ppm

MS (m/e): 140 ($M^+$), 125 (100%) 112, 97, 83, 79, 69, 55, 43.

Beispiel 8

a) 67,99 g (492 mMol) Kaliumcarbonat und 1,87 g (8,2 mMol) Benzyltriäthylammoniumchlorid werden in 500 ml Acetonitril suspendiert. Unter Rühren gibt man 59,86 g (410 mMol) Methyl-4-methoxyacetoacetat zu. Innert 30 Minuten tropft man 65,50 g (615 mMol) 1-Chlor-2-butanon zu und rührt das Gemisch 23 Stunden bei 20—25°C. Das Reaktionsgemisch wird auf 1 l 1N HCl gegossen (pH-Wert = 1) und es wird 3 mal mit je 700 ml Aethylacetat extrahiert. Die vereinigten organischen Phasen werden über Magnesiumsulfat getrocknet und am Rotationsverdampfer eingeengt. Das Rohprodukt destilliert bei 115°C/0,05 mbar. Es resultieren 49,01 g (55,3%) Methyl-4-methoxy-2-(2-oxobutyl)-acetoacetat.

IR (fl. Film): $1740^s$, $1720^s$, $1630^m$ $cm^{-1}$

NMR ($CDCl_3$) 60MHz: 1,08 (t/3H), 2,51 (q/2H), 3,12 (m/2H), 3,48 (s/3H), 3,78 (s/3H), 4,15 (m/1H), 4,30 (s/2H) ppm

MS (m/e): 216 ($M^+$), 184, 171, 139, 57, 45 (100%).

b) 21,60 g (100 mMol) Methyl-4-methoxy-2-(2-oxobutyl)-acetoacetatwerden mit 212,0 g (50 mMol) 2,5%-iger $Na_2CO_3$-Lösung während 8 Stunden bei Rückflusstemperatur gehalten. Das Reaktionsgemisch wird nach Abkühlen 3 mal mit je 200 ml $CH_2Cl_2$ extrahiert. Die vereinigten organischen Phasen werden über Magnesiumsulfat getrocknet und am Rotationsverdampfer eingeengt. Das Rohprodukt wird über $SiO_2$ säulenchromatographiert und ergibt 4,08 g (29,1%) 3-Aethyl-2-methoxy-2-cyclopenten-1-on.

IR (fl. Film): $1700^s$, $1635^s$ $cm^{-1}$

NMR ($CDCl_3$) 60MHz: 1,13 (t/3H), 2,43 (s/4H), 2,46 (q/2H), 3,93 (s/3H) ppm

MS (m/e): 140 ($M^+$, 100%), 125, 111, 97, 69.

c) 3,08 g (22 mMol) 3-Aethyl-2-methoxy-2-cyclopenten-1-on werden mit 30,80 g 5N Salzsäure während 5 Stunden bei Rückflusstemperatur gehalten. Das Reaktionsgemisch wird nach Abkühlen 3 mal mit je 50 ml $CH_2Cl_2$ extrahiert. Die vereinigten organischen Phasen werden über Magnesiumsulfat getrocknet und am Rotationsverdampfer eingeengt. Das Rohprodukt wird bei 0,1 mBar/110°C kugelrohrdestilliert und ergibt 1,82 g (65,7%) 3-Aethyl-2-hydroxy-2-cyclopenten-1-on (Schmelzpunkt 39—41°C).

IR ($CHCl_3$): $3500^m$, $3310^m$ (breit), $1710^s$, $1660^s$ $cm^{-1}$

NMR ($CDCl_3$) 60MHz: 1,13 (t/3H), 2,43 (s/4H), 2,46 (q/2H), 6,60 (s, breit/1H) ppm

MS (m/e): 126 ($M^+$, 100%), 111, 77, 83, 69, 55, 43.

Beispiel 9

a) 22,80 g (165 mMol) Kaliumcarbonat und 0,69 g (3 mMol) Benzyltriäthylammoniumchlorid werden in 150 ml Acetonitril suspendiert. Unter Rühren gibt man 32,42 g (150 mMol) Methyl-4-methoxy-2-(1-methylacetonyl)-acetoacetat hinzu und erwärmt auf 60°C. Innert 100 Minuten tropft man 28,08 g (180 mMol) Aethyljodid zu und rührt das Gemisch noch 90 Stunden bei 60°C. Dann giesst man die Suspension auf eine Lösung von 10,35 g (75 mMol) Kaliumcarbonat in 450 ml Wasser und erhitzt 30 Stunden bei Rückflusstemperatur. Die erkaltete Reaktionslösung wird 3 mal mit 300 ml $CH_2Cl_2$ extrahiert. Die vereinigten organischen Phasen werden über Magnesiumsulfat getrocknet und am Rotationsverdampfer eingeengt. Das Rohprodukt wird bei 20 mbar/97—99°C destilliert und ergibt 3,61 g (14,3%) 5-Aethyl-2-methoxy-3,4-dimethyl-2-cyclopenten-1-on.

IR (fl. Film): $1700^s$, $1640^2$ $cm^{-1}$

NMR ($CDCl_3$) 60MHz: 0,82—1,10 (m/3H), 2,20 (d/3H), 1,94 (s/3H) 1,32—2,61 (m/4H) 3,93 (s/3H) ppm

MS (m/e): 168 ($M^+$), 153, 140, 125, 108 (100%), 93, 55.

b) 2,06 g (12,2 mMol) 5-Aethyl-2-methoxy-3,4-dimethyl-2-cyclopenten-1-on werden mit 20,6 g 5N Salzsäure während 2 Stunden bei Rückflusstemperatur gehalten. Die erkaltete Reaktionslösung wird 3 mal mit 50 ml $CH_2Cl_3$ extrahiert.

Die vereinigten organischen Phasen werden über Magnesiumsulfat getrocknet und am Rotationsverdampfer eingeengt. Das Rohprodukt wird bei 1 mbar/104°C kugelrohrdestilliert und ergibt 1,43 g (76,1%) eines Gemisches von 5-Aethyl-2-hydroxy-3,4-dimethyl-2-cyclopenten-1-on (Isomer A) und 3-Aethyl-2-hydroxy-4,5-dimethyl-2-cyclopenten-1-on (Isomer B) im Verhältnis von ca. 4:1. Gehalt (GC): Isomer A, 68,9%, Isomer B 17,1%.

IR (fl. Film): $3500^m$, $3300^m$ (breit), $1705^s$, $1655^s$ $cm^{-1}$

NMR ($CDCl_3$) 60MHz: 0,82—1,10 (m/ca. 3H), 2,20 (d/ca. 4H), 1,99 (s/ca. 3H), 1,32—2,92 (m/ca. 3H), 6,61 (s, breit/1H) ppm

MS (m/e): 154 ($M^+$), 139, 126, 108, 55, 43 (100%).

In den folgenden Beispielen steht "Verbindung I" für das neue 2-Hydroxy-3,4,5-trimethyl-2-cyclopenten-1-on.

Beispiel 10

Ein Mandel-Haselnuss-Aroma kann wie folgt zusammengesetzt sein:

| | Gewichtsteile | |
|---|---|---|
| Vanillin | 25,0 | 25,0 |
| Acetylpyrazin | 2,5 | 2,5 |
| Diacetyl | 3,0 | 3,0 |
| Dimethylresorcin | 3,0 | 3,0 |
| Furfural | 4,0 | 4,0 |
| Benzaldehyd | 8,0 | 8,0 |
| gamma-Nonalakton | 1,0 | 1,0 |
| Trimethylpyrazin | 1,0 | 1,0 |
| Propylenglykol | 922,5 | 946,5 |
| Corylone | 30,0 | — |
| Verbindung I | — | 6,0 |
| | 1000,0 | 1000,0 |

Dosierung in Milchdrink: 50 gr/100 Liter (eine analoge Dosierung ergibt sich für ein Ahorn-, Schokolade- und Kaffeearoma, etc.).

Die neue Verbindung I erbringt in Aromen sensorisch dieselben Effekte, wie ca. die 5-fache Menge an "Corylone". (Wie oben gesagt, ist die neue Verbindung, rein von der Geschmackstärke her betrachtet, sogar 20 mal stärker).

Beispiel 11

Tabak-Base

| | Gewichtsteile |
|---|---|
| a) o-tert.Butylcyclohexylacetat | 400 |
| Jasminöl synth. | 300 |
| Ketonmoschus | 40 |
| Sandela Givaudan | 40 |
| Styrallylacetat | 30 |
| Cumarin | 20 |
| Isobutylchinolin 10% DPG (Dipropylenglykol) | 10 |
| Lavendelöl | 10 |
| Vetiveröl | 10 |
| Galbanumöl | 10 |
| Vassuraöl | 10 |
| DPG | 40 |
| | 920 |

Gibt man zu obiger base 10 Teile einer 10%-igen Lösung in Aethanol der Verbindung I, so erhält die Komposition eine angenehme Wärme, ihre süsse Jasmin-Note wird durch eine frisch-herbe Note ergänzt.

b)

Leder-Base

| | Gewichtsteile |
|---|---|
| Styrax nat. RIFM | 250 |
| Castoreum-anhydrol | 150 |
| Bergamotteöl | 100 |
| Moschus inf. 3% in Aethanol | 100 |
| Vetiveröl | 100 |
| Labdanum-resinoid | 100 |
| Birkenteeröl dephenolisiert 10% DPG | 50 |
| Ketonmoschus | 25 |
| Sandelholzöl | 10 |
| Vanillin | 5 |
| Ciste Labdanum | 5 |
| DPG | 60 |
| | 960 |

Gibt man 10 Teile einer 10%-igen Lösung der Verbindung I zu obiger Base, so gewinnt die Leder-base

überraschenderweise eine starke rauchige Note, die sich sehr gut mit dem Ledercharakter der Base verbindet und diese harmonisch ergänzt, ohne dass das Birkenteeröl in unangenehmer Weise hervortritt.

c) Fougère-Base

| | Gewichtsteile |
|---|---|
| Lavendelöl | 200 |
| Linalylacetat | 150 |
| Baum-Moos absolu | 60 |
| Cumarin | 50 |
| Patchouliöl | 30 |
| Rhodinol extra ex Geraniumöl | 30 |
| Methyldihydrojasmonat | 30 |
| Ketonmoschus | 30 |
| Vetivenylacetat | 30 |
| Geranium BB synth. | 30 |
| Amylsalicylat | 20 |
| Sandela Givaudan | 20 |
| Linalool | 20 |
| Benzylacetat | 15 |
| Ylang-Ylangöl | 15 |
| Eugenol | 15 |
| Thymianöl | 5 |
| DPG | 50 |
| | 800 |

Gibt man 10 Teile einer 10%-igen Lösung der Verbindung I zu obiger Base, so wird die krautige Note der Fougère-Base in angenehmer Weise unterstrichen und die Lavendel-Note fein abgerundet.

d) Rosen-Base

| | Gewichtsteile |
|---|---|
| Phenyläthylalkohol | 300 |
| Geraniol | 250 |
| Jasmin "lavage" | 200 |
| Citronellol extra | 100 |
| alpha-Jonon | 40 |
| C-10-Aldehyd 10% DPG | 5 |
| C-11-Aldehyd 10% DPG | 5 |
| | 900 |

Gibt man 10 Teile einer 10%-igen Lösung der Verbindung I zu dieser Base, so erhält die Komposition

eine neuartige, interessante Richtung, sie wirkt nun sehr viel herber, ohne aber an Natürlichkeit einzübussen.

In obigem Beispiel wurde zudem die Verbindung I jeweils durch dieselbe Menge des bekannten 2-Hydroxy-3,4,4-trimethyl-2-cyclopenten-1-ons ersetzt. In allen diesen Fällen wurde ein negativer Effekt erzielt. In allen Fällen drang eine unangenehme Liebstöckelnote des bekannten Trimethylderivats durch, die den Kompositionen eine nicht erwünschte "Lebensmittel-Note" verlieh.

**Patentansprüche**

1. Verfahren zur Herstellung von Verbindungen der Formel

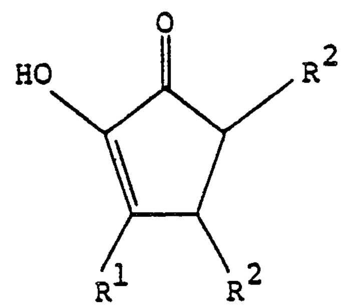

I

worin $R^1$ $C_{1-5}$-Alkyl, insbesondere Methyl, Aethyl. Propyl oder Isopropyl bedeutet und die Radikale $R^2$ unabhängig voneinander Wasserstoff oder $C_{1-5}$-Alkyl, insbesondere Wasserstoff oder Methyl, Aethyl, Propyl oder Isopropyl darstellen,
dadurch gekennzeichnet, dass man eine Verbindung der Formel

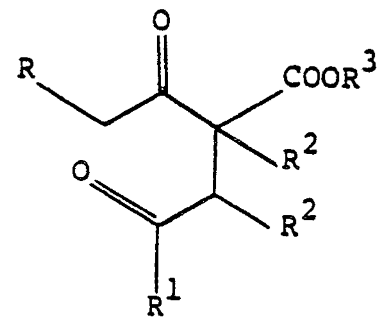

II

worin R für $C_{1-4}$-Alkoxy, Chlor, Brom oder $C_{1-4}$-Alkanoyloxy steht, $R^1$ und $R^2$ obige Bedeutung besitzen und $R^3$ $C_{1-4}$-Alkyl darstellt,
hydrolysiert und einer Aldolkondensation unterwirft, und, im Falle von R = $C_{1-4}$-Alkoxy, das Reaktionsprodukt anschliessend noch einer Säurebehandlung unterwirft.

2. Verfahren nach Anspruch 1, dadurch gekennzeichnet, dass R Methoxy darstellt.

3. Verfahren nach Anspruch 1, dadurch gekennzeichnet, dass R Acetoxy darstellt.

5. Verfahren nach einem der Ansprüche 1 bis 4, dadurch gekennzeichnet, dass man eine Verbindung der Formel II durch Alkylierung einer Verbindung der Formel

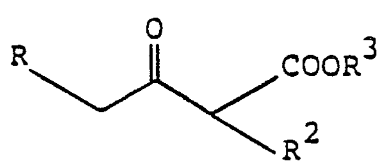

III

worin R, $R^2$ und $R^3$ obige Bedeutung besitzen, mittels einer Verbindung der Formel

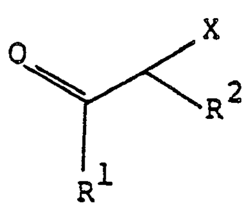

IV

worin $R^1$ und $R^2$ obige Bedeutung besitzen, und X Chlor oder Brom darstellt, gewinnt.

6. Verfahren nach einem der Ansprüche 1—5, dadurch gekennzeichnet, dass man von einer

Verbindung der Formel III mit $R^2$ = Wasserstoff ausgeht und die Verbindung der Formel II zunächst $C_{1-5}$-alkyliert.

7. Verfahren nach einem der Ansprüche 1 bis 6, dadurch gekennzeichnet, dass man 2-Hydroxy-3-methyl-2-cyclopenten-1-on herstellt.

8. Verfahren nach einem der Ansprüche 1 bis 6, dadurch gekennzeichnet, dass man 2-Hydroxy-3,4-dimethyl-2-cyclopenten-1-on herstellt.

9. Verfahren nach einem der Ansprüche 1 bis 6, dadurch gekennzeichnet, dass man 2-Hydroxy-3,5-dimethyl-2-cyclopenten-1-on herstellt.

10. Verfahren nach einem der Ansprüche 1 bis 6, dadurch gekennzeichnet, dass man 2-Hydroxy-3-äthyl-5-methyl-2-cyclopenten-1-on herstellt.

11. Verfahren nach einem der Ansprüche 1 bis 6, dadurch gekennzeichnet, dass man 2-Hydroxy-5-äthyl-3-methyl-2-cyclopenten-1-on herstellt.

12. Verfahren nach einem der Ansprüche 1 bis 6, dadurch gekennzeichnet, dass man 2-Hydroxy-3-äthyl-2-cyclopenten-1-on herstellt.

13. Verfahren nach einem der Ansprüche 1 bis 6, dadurch gekennzeichnet, dass man 2-Hydroxy-3,4,5-trimethyl-2-cyclopenten-1-on herstellt.

14. Verfahren nach einem der Ansprüche 1 bis 6, dadurch gekennzeichnet, dass man 2-Hydroxy-3-äthyl-4,5-dimethyl-2-cyclopenten-1-on herstellt.

15. Verfahren nach einem der Ansprüche 1 bis 6, dadurch gekennzeichnet, dass man 2-Hydroxy-5-äthyl-3,4-dimethyl-2-cyclopenten-1-on herstellt.

16. Eine Verbindung, ausgewählt aus 2-Hydroxy-3,4,5-trimethyl-2-cyclopenten-1-on, 2-Hydroxy-3-äthyl-4,5-dimethyl-2-cyclopenten-1-on und 2-Hydroxy-5-äthyl-3,4-dimethyl-2-cyclopenten-1-on.

17. Riech- und/oder Geschmackstoffkompositionen, gekennzeichnet durch einen Gehalt an einer Verbindung gemäss Anspruch 16.

18. Verwendung einer Verbindung gemäss Anspruch 16 als Riech- und/oder Geschmackstoff.

19. Verbindungen der Formel

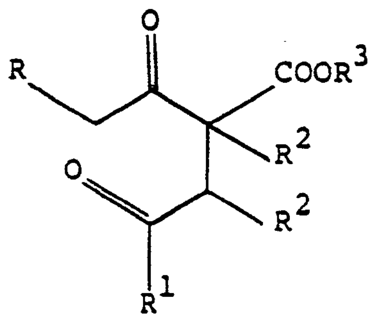

II

worin R $C_{1-4}$-Alkoxy, Chlor, Brom oder $C_{1-4}$-Alkanoyloxy darstellt, $R^1$ $C_{1-5}$-Alkyl, insbesondere Methyl, Aethyl, Propyl oder Isopropyl bedeutet, die Radikale $R^2$ unabhängig voneinander Wasserstoff oder $C_{1-5}$-Alkyl, insbesondere Wasserstoff oder Methyl, Aethyl, Propyl oder Isopropyl darstellen und $R^3$ $C_{1-4}$-Alkyl bedeutet.

20. Methyl-4-methoxy-2-acetonylacetoacetat.

21. Methyl-4-acetoxy-2-acetonylacetoacetat.

22. Methyl-4-chlor-2-acetonylacetoacetat.

23. Methyl-4-methoxy-2-[1-methylacetonyl]-acetoacetat.

24. Methyl-4-methoxy-2-acetonyl-2-methyl-acetoacetat.

25. Methyl-4-methoxy-2-(2-oxobutyl)-acetoacetat.

26. 2-Methoxy-3,4,5-trimethyl-2-cyclopenten-1-on.

27. 5-Aethyl-2-methoxy-3,4-dimethyl-2-cyclopenten-1-on.

## Revendications

1. Procédé de préparation de composés de formule

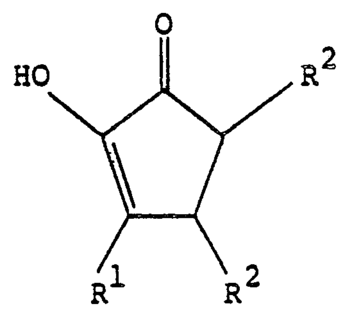

I

dans laquelle $R^1$ représente un groupe alkyle en $C_1$—$C_5$, en particulier un groupe méthyle, éthyle, propyle ou isopropyle, et les symboles $R^2$ représentent chacun, indépendamment les uns des autres, l'hydrogène ou un groupe alkyle en $C_1$—$C_5$, en particulier l'hydrogène ou un groupe méthyle, éthyle, propyle ou isopropyle,
caractérisé en ce que l'on hydrolyse et on soumet à une condensation aldolique un composé de formule

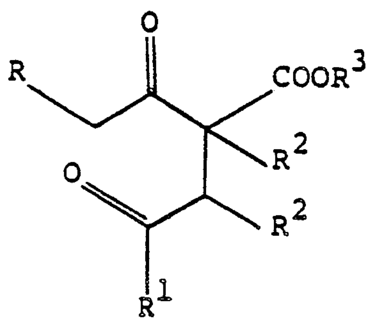

II

dans laquelle R représente un groupe alcoxy en $C_1$—$C_4$, le chlore, le brome ou un groupe alcanoyloxy en $C_1$—$C_4$, $R^1$ et $R^2$ ont les significations indiquées ci-dessus et $R^3$ représente un groupe alkyle en $C_1$—$C_4$, et, dans le cas où R représente un groupe alcoxy en $C_1$—$C_4$, on soumet ensuite le produit de réaction à un traitement acide.

2. Procédé selon la revendication 1, caractérise en ce que R représente un groupe méthoxy.

3. Procédé selon la revendication 1, caractérisé en ce que R représente le chlore.

4. Procédé selon la revendication 1, caractérisé en ce que R représente un groupe acétoxy.

5. Procédé selon l'une des revendications 1 à 4, caractérisé en ce que l'on obtient un composé de formule II par alkylation d'un composé de formule

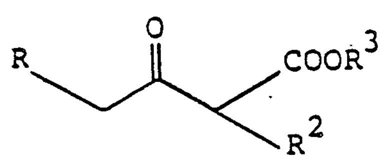

III

dans laquelle R, $R^2$ et $R^3$ ont les significations indiquées ci-dessus,
à l'aide d'un composé de formule

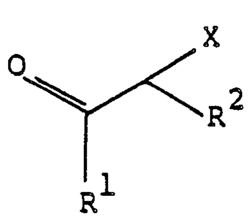

IV

dans laquelle $R^1$ et $R^2$ ont les significations indiquées ci-dessus et X représente le chlore ou le brome.

6. Procédé selon l'une des revendications 1 à 5, caractérisé en ce que l'on part d'un composé de

formule III dans laquelle $R^2$ représente l'hydrogène et on soumet d'abord le composé de formule II à une alkylation en $C_1$—$C_5$.

7. Procédé selon l'une des revendications 1 à 6, caractérisé en ce que l'on prépare la 2-hydroxy-3-méthyl-2-cyclopentène-1-one.

8. Procédé selon l'une des revendications 1 à 6, caractérisé en ce que l'on prépare la 2-hydroxy-3,4-diméthyl-2-cyclopentène-1-one.

9. Procédé selon l'une des revendications 1 à 6, caractérisé en ce que l'on prépare la 2-hydroxy-3,5-diméthyl-2-cyclopentène-1-one.

10. Procédé selon l'une des revendications 1 à 6, caractérisé en ce que l'on prépare la 2-hydroxy-3-éthyl-5-méthyl-2-cyclopentène-1-one.

11. Procédé selon l'une des revendications 1 à 6, caractérisé en ce que l'on prépare la 2-hydroxy-5-éthyl-3-méthyl-2-cyclopentène-1-one.

12. Procédé selon l'une des revendications 1 à 6, caractérisé en ce que l'on prépare la 2-hydroxy-3-éthyl-2-cyclopentène-1-one.

13. Procédé selon l'une des revendications 1 à 6, caractérisé en ce que l'on prépare la 2-hydroxy-3,4,5-triméthyl-2-cyclopentène-1-one.

14. Procédé selon l'une des revendications 1 à 6, caractérisé en ce que l'on prépare la 2-hydroxy-3-éthyl-4,5-diméthyl-2-cyclopentène-1-one.

15. Procédé selon l'une des revendications 1 à 6, caractérisé en ce que l'on prépare la 2-hydroxy-5-éthyl-3,4-diméthyl-2-cyclopentène-1-one.

16. Un composé choisi parmi la 2-hydroxy-3,4,5-triméthyl-2-cyclopentène-1-one, la 2-hydroxy-3-éthyl-4,5-diméthyl-2-cyclopentène-1-one, et la 2-hydroxy-5-éthyl-3,4-dimethyl-2-cyclopentène-1-one.

17. Compositions odorantes et/ou aromatiques caractérisées en ce qu'elles contiennent un composé selon la revendication 16.

18. Utilization d'un composé selon la revendication 16 en tant que substance odorante et/ou aromatique.

19. Composé de formule

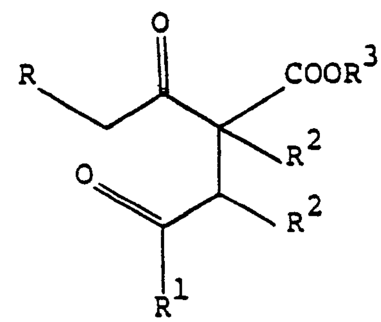

II

dans laquelle R représente un groupe alcoxy en $C_1$—$C_4$, le chlore, le brome ou un groupe alcanoyloxy en $C_1$—$C_4$, $R^1$ représente un groupe alkyle en $C_1$—$C_5$, en particulier un groupe méthyle, éthyle, propyle ou isopropyle, les symboles $R^2$ représentent chacun, indépendamment l'un de l'autre, l'hydrogène ou un groupe alkyle en $C_1$—$C_5$, en particulier l'hydrogène ou un groupe méthyle, éthyle, propyle ou isopropyle, et $R^3$ représente un groupe alkyle en $C_1$—$C_4$.

20. Le 4-méthoxy-2-acétonylacétoacétate de méthyle.

21. Le 4-acétoxy-2-acétonylacétoacétate de méthyle.

22. Le 4-chloro-2-acétonylacétoacétate de méthyle.

23. Le 4-méthoxy-2-(1-méthylacétonyl)-acétoacétate de méthyle.

24. Le 4-méthoxy-2-acétonyl-2-méthyl-acétoacétate de méthyle.

25. Le 4-méthoxy-2-(2-oxobutyl)-acétoacétate de méthyle.

26. La 2-méthoxy-3,4,5-triméthyl-2-cyclopentène-1-one.

27. La 5-éthyl-2-méthoxy-3,4-diméthyl-2-cyclopentène-1-one.

## Claims

1. A process of the manufacture of compounds of the formula

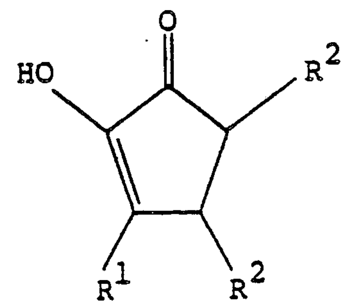

I

wherein $R^1$ signifies $C_{1-5}$-alkyl, especially methyl, ethyl, propyl or isopropyl, and the radicals $R^2$ each independently represent hydrogen or $C_{1-5}$-alkyl, especially hydrogen or methyl, ethyl, propyl or isopropyl, characterized in that a compound of the formula

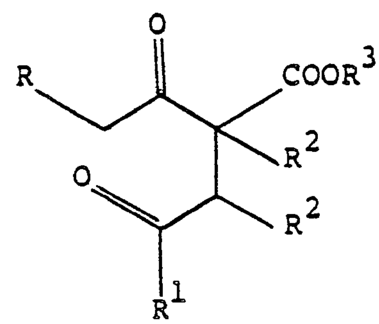

II

wherein R stands for $C_{1-4}$-alkoxy, chlorine, bromine or $C_{1-4}$-alkanoyloxy, $R^1$ and $R^2$ have the above significance and $R^3$ represents $C_{1-4}$-alkyl, is hydrolyzed and subjected to an aldol condensation and, where R = $C_{1-4}$-alkoxy, the reaction product is subsequently subjected to an acid treatment.

2. A process according to claim 1, characterized in that R represents methoxy.

3. A process according to claim 1, characterized in that R represents chlorine.

4. A process according to claim 1, characterized in that R represents acetoxy.

5. A process according to any one of claims 1 to 4, characterized in that a compound of formula II is produced by alkylating a compound of the formula

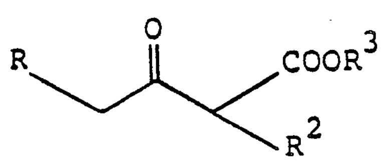

III

wherein R, $R^2$ and $R^3$ have the above significance, by means of a compound of the formula

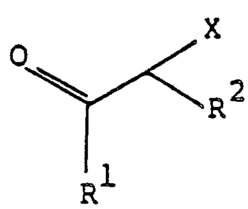

IV

wherein $R^1$ and $R^2$ have the above significance and X represents chlorine or bromine.

6. A process according to any one of claims 1—5, characterized in that a compound of formula III with $R^2$ = hydrogen is used and the compound of formula II is firstly $C_{1-5}$-alkylated.

7. A process according to any one of claims 1 to 6, characterized in that 2-hydroxy-3-methyl-2-cyclopenten-1-one is manufactured.

8. A process according to any one of claims 1 to 6, characterized in that 2-hydroxy-3,4-dimethyl-2-cyclopenten-1-one is manufactured.

9. A process according to any one of claims 1 to 6, characterized in that 2-hydroxy-3,5-dimethyl-2-cyclopenten-1-one is manufactured.

10. A process according to any one of claims 1 to 6, characterized in that 2-hydroxy-3-ethyl-5-methyl-2-cyclopenten-1-one is manufactured.

11. A process according to any one of claims 1 to 6, characterized in that 2-hydroxy-5-ethyl-3-methyl-2-cyclopenten-1-one is manufactured.

12. A process according to any one of claims 1 to 6, characterized in that 2-hydroxy-3-ethyl-2-cyclopenten-1-one is manufactured.

13. A process according to any one of claims 1 to 6, characterized in that 2-hydroxy-3,4,5-trimethyl-2-cyclopenten-1-one is manufactured.

14. A process according to any one of claims 1 to 6, characterized in that 2-hydroxy-3-ethyl-4,5-dimethyl-2-cyclopenten-1-one is manufactured.

15. A process according to any one of claims 1 to 6, characterized in that 2-hydroxy-5-ethyl-3,4-dimethyl-2-cyclopenten-1-one is manufactured.

16. A compound selected from 2-hydroxy-3,4,5-trimethyl-2-cyclopenten-1-one, 2-hydroxy-3-ethyl-4,5-dimethyl-2-cyclopenten-1-one and 2-hydroxy-5-ethyl-3,4-dimethyl-2-cyclopenten-1-one.

17. Odorant and/or flavourant compositions, characterized by a content of a compound in accordance with claim 16.

18. The use of a compound in accordance with claim 16 as an odorant and/or flavourant.

19. Compounds of the formula

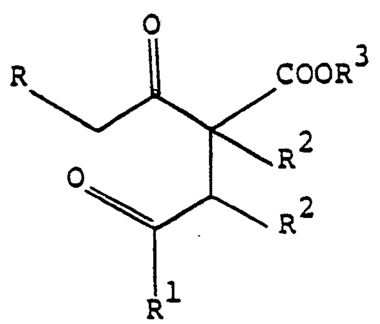

II

wherein R represents $C_{1-4}$-alkoxy, chlorine, bromine or $C_{1-4}$-alkanoyloxy, $R^1$ signifies $C_{1-5}$-alkyl, especially methyl, ethyl, propyl or isopropyl, the radicals $R^2$ each independently represent hydrogen or $C_{1-5}$-alkyl, especially hydrogen or methyl, ethyl, propyl or isopropyl, and $R^3$ signifies $C_{1-4}$-alkyl.

20. Methyl 4-methoxy-2-acetonylacetoacetate.

21. Methyl 4-acetoxy-2-acetonylacetoacetate.

22. Methyl 4-chlaoro-2-acetonylacetoacetate.

23. Methyl 4-methoxy-2-[1-methylacetonyl]-acetoacetate.

24. Methyl 4-methoxy-2-acetonyl-2-methyl-acetoacetate.

25. Methyl 4-methoxy-2-(2-oxobutyl)-acetoacetate.

26. 2-Methoxy-3,4,5-trimethyl-2-cyclopenten-1-one.

27. 5-Ethyl-2-methoxy-3,4-dimethyl-2-cyclopenten-1-one.